(19)

Europäisches
Patentamt
European
Patent Office
Office européen
des brevets

(11) **EP 3 636 141 A1**

(12) **EUROPEAN PATENT APPLICATION**

(43) Date of publication:
**15.04.2020 Bulletin 2020/16**

(21) Application number: **18199737.0**

(22) Date of filing: **10.10.2018**

(51) Int Cl.:
*A61B 5/00* (2006.01)     *A61B 5/145* (2006.01)
*A61B 5/1455* (2006.01)     *A61B 5/1495* (2006.01)
*G01N 21/35* (2014.01)     *G01N 33/66* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Prediktor Medical AS**
**1630 Fredrikstad (NO)**

(72) Inventors:
• **Karstang, Terje**
  **Fredrikstad (NO)**
• **Schoenfelder, Sven**
  **Heilbronn (DE)**

(74) Representative: **Gill Jennings & Every LLP**
**The Broadgate Tower**
**20 Primrose Street**
**London EC2A 2ES (GB)**

(54) **WEARABLE BLOOD GLUCOSE SENSOR**

(57)     A wearable device for estimating the blood glucose level of the wearer, the device including a source of near-infrared radiation arranged to irradiate a measurement area of the skin of the wearer; a spectrometer arranged to receive reflected or transmitted near infrared radiation and output an absorption spectrum comprising the absorption intensity at a plurality of wavelengths, wherein all of the wavelengths are within a portion of the near infrared spectrum having a substantially constant optical penetration depth; a processing unit configured to: receive the absorption spectrum from the spectrometer; calculate an estimate of the blood glucose level of the wearer using a stored calibration model describing the relationship between near infrared absorption and blood glucose level.

**EP 3 636 141 A1**

## Description

### TECHNICAL FIELD

The present invention relates to a wearable device for non-invasive sensing of a blood glucose level of the wearer.

### BACKGROUND

A great deal of control systems operate on the basis of knowing the levels of various substances within a material, often where the material is made of multiple different substances. This is typically measured by taking a sample of the material. This sample is then used in various chemical and physical tests to determine its composition. Such direct measurements may be termed "invasive" methods.

However, in some situations it is undesirable or infeasible to directly measure the level of the substance in the material by taking a sample. This may be because of the cost, difficulty or danger of retrieving the samples.

A further difficulty arises when the level of the substance changes over time, for example due to metabolism or decay. This necessitates continually taking invasive samples in order to have accurate information about the level. However, it may be undesirable to repeatedly take samples of the material for conventional analysis, as taking such samples may damage the material, and the analysis tends to destroy the sample.

In particular, many modern medical techniques rely on knowing the concentration of a chemical constituent in blood. For example, people with diabetes need to regularly monitor their blood glucose levels. Conventionally, this would involve taking hourly blood samples from the person using invasive means. For example, a needle may be inserted into a vein in the arm of the person, and a sample of the blood extracted into a syringe or by using a finger pricking device. This sample can then be analysed directly by a suitable device. This process has the advantage of providing a high accuracy reading of the blood glucose level. However, in practice there are many disadvantages of using such invasive means. In particular, they are inconvenient and tend to require significant training to safely use. Studies have shown that 60% of respondents have skipped measuring their glucose levels due to the invasive nature of conventional testing. More problematically, even when used properly, taking blood samples may cause tissue damage, and can result in infection.

Because of these disadvantages, there has been research into non-invasive methods for taking samples. For example, the use of optical and/or bio-impedance sensors may provide an estimate of the level of glucose in the blood. However, while such non-invasive methods are more convenient, they tend to be of low accuracy. One of the reasons for the low accuracy is that such sensors tend to measure not only the blood, but also other undesired tissues in the body. The accuracy of the results is reduced further by the fact that the dermis is very heterogenic. Furthermore, the models used to relate the measured signal with glucose level are sensitive to a number of parameters such as temperature, pressure, the amount of moisture for example from sweat, cooling effects introduced by the device and different trends between day and night data.

A further important and related issue is that the user's movement introduces significant artefacts (motion artefacts - MAs) into the reading which prevent known devices from providing a measurement of sufficient accuracy.

In a laboratory setting motion and other parameters can be carefully controlled such that fairly accurate results can be obtained where the conditions are closely monitored and controlled. However, such devices must be workable outside of the laboratory for the user to gain the full benefits. The above described issues have thus far prevented the development of a non-invasive sensing device which can provide a sufficiently accurate measurement of blood glucose outside of the laboratory, where external parameters such as temperature, pressure and motion cannot be controlled.

There is accordingly a need for a sensor which makes progress in overcoming some of the above issues in known devices to provide a non-invasive glucose monitoring device which can provide measurements of increased accuracy outside of the laboratory. There is a particular need for a device which can make progress in overcoming motion artefacts.

### SUMMARY OF INVENTION

In a first aspect, there is provided a wearable device for estimating the blood glucose level of the wearer, the device comprising: a source of near-infrared radiation arranged to irradiate a measurement area of the skin of the wearer; a spectrometer arranged to receive reflected or transmitted near infrared radiation and output an absorption spectrum comprising the absorption intensity at a plurality of wavelengths, wherein all of the wavelengths are within a portion of the near infrared spectrum having a substantially constant optical penetration depth; a processing unit configured to: receive the absorption spectrum from the spectrometer; calculate an estimate of the blood glucose level of the wearer using a stored calibration model describing the relationship between near infrared absorption and blood glucose level.

By using this device, motion artefacts can be considerably reduced and a more accurate blood glucose measurement can be provided. This is because by using a portion of the NIR spectrum with a substantially constant optical penetration depth, each wavelength of light emitted follows substantially the same path in the tissue and therefore the corresponding absorption readings correspond to the same area of tissue. In comparison, known devices use broader ranges of wavelengths of which the penetration depth varies significantly. In such devices each measurement (at a different wavelength) corresponds to a different position in the tissue which introduces significant errors into the results. By limiting the portion of the NIR absorption spectra used to estimate blood glucose level to a portion with a substantially constant and preferably low optical penetration depth, errors due to motion artefacts and sampling of different tissue composition are significantly reduced. The wearable device according to the present invention can therefore achieve increased accuracy in blood glucose level estimates outside of the laboratory.

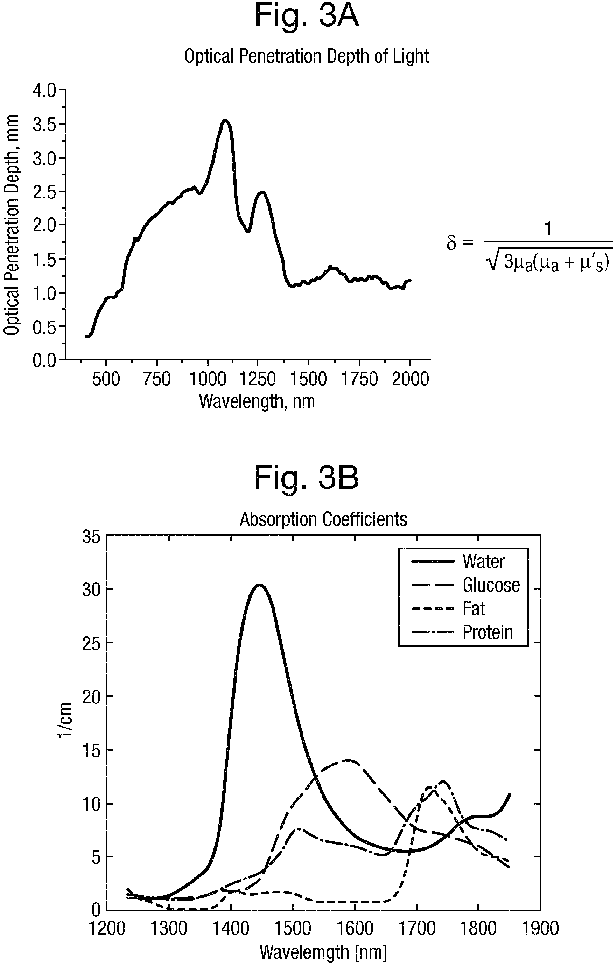

The phrase "substantially constant optical penetration depth" refers to the penetration depth of the near infrared radiation in human skin and/or tissue. "Substantially constant" may be defined as a variation of less than 25% across the portion of the near infrared spectrum, preferably less than 15%, or more preferably less than 10% of the maximum penetration depth within the portion of the spectrum selected. "Substantially constant" may also be defined as changing by less than 1.0 mm, preferably less than 0.5 mm, more preferably less than 0.25 mm across the chosen portion of the near infrared spectrum. As shown in Figure 3A the optical penetration depth is substantially constant between around 1350 nm to 2000 nm.

The phrase "wearable device" is intended to encompass any device which can be retained in use to part of the body of a user. It includes a watch, any form of strap or patch, any item of clothing which can incorporate the device such that it is held adjacent to the skin so that it can function. The device may also be provided as a standalone sensor which is "wearable" in use, for example by a user strapping the sensor to their body using separate components, i.e. it need not include any components which allow it to be attached to a user but may simply be a sensor which, in use, can be arranged to collect data from the skin as defined in claim 1.

Preferably the device is attached to the arm of a user by half-circle flexible hoop connecting two housings, one housing comprising the spectrometer, light source and processor and the other housing a power source. This arrangement naturally balances the weight of the sensor components in the first housing with the battery in the second housing.

Preferably the selected portion of the near infrared spectrum having a substantially constant optical penetration depth is in the range 1400 to 1800 nm. In particular, the absorption spectra may be limited to falling within this wavelength range, i.e. absorption at wavelengths outside of this range is not measured and/or used for estimating glucose. This portion of the NIR spectrum has a low and substantially constant optical penetration depth in the skin and therefore the absorption spectra relate to substantially the same measured volume of the tissue, thus increasing the accuracy of the reading in comparison to other spectral ranges in which the optical paths substantially vary by wavelength. In other words by limiting the measured absorption to wavelengths in this range it is ensured that all wavelengths measure at substantially the same depth (and therefore volume). Furthermore, because light in the wavelength range of 1400 to 1800 nm has a low penetration depth, readings are less affected by movement since the volume defined by the changing orientation of the incident light is reduced at lower penetration depths. A further benefit is that this portion of the spectrum encompasses the absorption peak of glucose, thus providing a stronger signal with which the glucose percentage can be estimated.

In some examples a narrower range may be used, for example 1500 nm to 1800 nm which more closely bounds the absorption peak of glucose or a more specific range still such as around 1550 nm to 1650 nm to further select the extreme peak of the glucose response, whilst still remaining in a portion of the spectrum in which the penetration depth is substantially constant.

Preferably the spectrometer is arranged to measure the near infrared absorption spectrum over at least 10 wavelengths in the range 1400 to 1800 nm. This allows accurate modelling of the changes in temperature, pressure and concentration, providing a more reliable estimate of blood glucose level.

Preferably the wearable device includes a temperature sensor arranged to measure the temperature of the measurement area of the skin of the wearer; wherein the processing unit is further configured to: receive the temperature from the temperature sensor; and adjust the absorption spectra using a latent structure decomposition method using the measured temperature. Preferably the latent structure decomposition method is marker variable projection based on the measured temperature. The marker variable projection method is described in "Latent-Structure Decompositions (Projections) of Multivariate Data", O. M Kvalheim, Chemometrics and Intelligent Laboratory Systems, 2 (1987) 283-290.

In other embodiments the temperature of the measured area of the skin may be determined by the shift in position of the absorbance peak of water and therefore does not require a dedicated temperature sensor. Since the absorption peak of water varies significantly with temperature, this may be used to indicate the temperature of the measurement area of the skin.

The output of the source of near infrared radiation may also be calibrated based on the temperature of the source, for example as detected by the temperature sensor arranged to measure the temperature of the measurement area of the skin or via a dedicated temperature sensor arranged to measure the temperature of the source. The calibration may take the form of the method described in WO2017/129633. In particular, a source LED may be calibrated by determining a reference forward voltage, $V_{f0}$, a reference forward current, $I_{f0}$, and a reference detected intensity of emitted light, $S_0$, of the LED at an initial junction temperature, $T_0$; varying the junction temperature of the LED to provide a plurality of junction temperatures $T_j$ and at each junction temperature: setting the forward current to a plurality of forward current values $I_f$, and at each forward current: determining the deviation in the forward voltage $\Delta V_f$ from the reference voltage $V_{f0}$; determining the deviation in forward current $\Delta I_f$ from the reference current $I_{f0}$; and determining the deviation in detected light intensity $\Delta S$ from the reference intensity $S_0$; estimating the parameters $s_V$, $s_I$, $s_{IV}$, $s_{II}$, and $s_{VV}$ such that:

$$S = S_0 + \Delta S = S_0 + s_V \Delta V_f + s_I \Delta I_f + s_{IV} \Delta V_f \Delta I_f + s_{II} \Delta I_f^2 + s_{VV} \Delta V_f^2 \quad \text{approximately holds for all sets of for-}$$

ward voltage deviation, $\Delta V_f$, forward current deviation, $\Delta I_f$, and detected intensity deviation, $\Delta S$.

In this manner, a model of the relationship between the intensity of the detected light (which is proportional to the light emitted from the LED) and the LED forward voltage and current can be established (the former varying with junction temperature). Such a model can then be used to control the emitted output of an LED, or to correct the detected light intensity (transmitted by diffuse reflection or by transmission) for the effects of a change in temperature such that a first output and a second output can be compared without an intervening change in the environment limiting the accuracy of any such comparison.

The use of a constructed mathematical model to calibrate for changes in the operating environment of the LED does not require any calibration data to be stored in the device or to be accessed from a remote memory in order to calibrate the output during use. Since the model is non-linear it avoids any need for linear interpolation between stored calibration values and provides an increased precision. There is no requirement for the LED to undergo a special compensation procedure when calibrating or adjusting the LED output. For example the LED operating values do not have to be altered to measure a voltage or current during a calibration routine. In this method normal operating values are used during calibration and when using the calibrated LED such that normal operation of the LED does not have to be interrupted.

The deviation values, indicated by $\Delta$, are the changes in the measured values from some standard reference values ($V_0$, $I_0$, $S_0$) around which the modelling is carried out. For each deviation measurement in the forward voltage, $V_f$ and the LED current, $I_f$, the resulting measured intensity deviation $\Delta S$ from the standard value $S_0$ is measured as well.

The junction temperature is varied by varying the ambient temperature of the LED in steps. The junction temperature does not enter into the prediction model directly, but will cause changes in the forward voltage and current as described above. For model adaptation purposes, the ambient temperature, as well as the LED current, is varied independently during the calibration procedure. The LED current is controlled in standard ways by LED current control electronics which receives a set point for the LED forward current. Based on this procedure, where corresponding values of LED forward voltage and currents (inputs to the model), as well as registered light detection (output from the model) are detected, the model parameters can be estimated by minimizing the model prediction errors based on some objective function. The emitted light is measured by diffuse reflection from a standard reference material disc or otherwise (i.e. transmission).

In some embodiments the variations in ambient temperature and forward current span the planned operating range of the LEDs in terms of temperatures and LED currents. In particular, in some embodiments, the reference values $V_{f0}$, $I_{f0}$, $S_0$, $T_0$ are selected such that: the initial junction temperature, $T_0$, falls within a predefined expected operational temperature range; and the reference intensity, $S_0$, falls within a predetermined desired range for the intended operation.

In certain embodiments the detected reference light intensity $S_0$ is measured by diffuse reflection or transmission from a standard reference material and is proportional to the emitted light intensity. Estimating the parameters can be performed in any suitable way. However, in preferred embodiments, estimating the parameters comprises using a partial least squares regression analysis.

Preferably the processing unit is further configured to remove the effects of scattering in the measured intensities received from the spectrometer. For example, the processing unit may be configured to remove effects of scattering by calculating a second derivative of the absorption spectrum.

Preferably, the processing unit is configured to determine the calibration model by carrying out a calibration routine comprising: receiving the measured absorption spectrum during a calibration period; receiving actual blood glucose level measurement data, recorded at regular intervals during the calibration period; fitting the measured absorbance to the actual glucose level measurement data to determine the model describing the relationship between the near infrared absorption and blood glucose level. Preferably the processing unit is configured to fit the measured absorption to the actual glucose level measurement data by using partial least squares regression.

Preferably a calibration routine is carried out during an initial calibration period to calibrate the device to a specific user. The calibration routine preferably involves a user wearing the device during the calibration period in which absorbance spectra are continuously measured while conventional measurements of blood glucose level are taken, for example via invasive measurement such as finger prick testing. The calibration period may involve the user changing their blood sugar levels by consuming sugar, for example in the form of food or drink, or undergoing physical activities such as various forms of exercise. The activities may be aimed at creating different levels of blood pressure, pulse temperature and sensor skin pressure. These changes in blood sugar level during the calibration period may be measured via actual, i.e. invasive measurements of glucose at regular intervals, for example at a frequency of every 5 minutes. The data measured via conventional methods is then provided to the device, for example by user input or by other means of transfer such as wireless/cabled connection.

The calibration model may be determined from the measured spectra and the blood glucose readings for example using principal component regression model, a partial least squares regression model or other modelling approaches based on latent variables.

In particular the modelling may follow aspects of the latent variable approach described in WO2017/129634.

Although in preferred embodiments the calibration model is determined per user, the calibration model may equally be a global calibration model in which the calibration routine is carried out on multiple users to determine a calibration model which is applicable to all users. In this way the wearable device can be calibrated in the factory and the user does not need to carry out any calibration routine, increasing ease of use.

Preferably the calibration model is also determined solely using wavelengths in the range 1400 to 1800 nm. In this way, wavelengths which have a different optical penetration depth do not contribute to the model so that inaccuracies due to the incorporation of absorption signals from larger volumes are not introduced.

The absorption spectra data collected during the calibration period may be pre-processed by removing scattering effects and/or removing skin temperature effects as described above before fitting to the glucose levels measured by conventional means.

Preferably the absorption spectrum received during the calibration period are collected from a plurality of distinct locations on the skin of the wearer and the processing unit is further configured to use the spectrum from distinct areas to adapt the calibration model to account for natural background variations. In particular, if the calibration data is collected from a single measurement area of the skin then, if the device moves slightly, an offset will enter the readings due to the slightly different composition of the tissue sampled by the device in the new location. By collecting calibration data from a number of locations the natural background variations can be accounted for, for example by averaging to substantially remove offset seen if the device shifts slightly.

Preferably the wearable device of any preceding claim is configured to direct the illuminating near-infrared radiation to multiple distinct measurement areas of the skin, the spectrometer arranged to receive the reflected or transmitted near infrared radiation from the multiple areas to determine an average absorbance of a larger representative skin area. In this way a more accurate measure of the glucose level is determined by sampling a larger representative area of the skin. Similarly the calibration routine may be performed using NIR spectra measured from multiple distinct measurement areas which can be averaged before fitting to the actual measurements of glucose level obtained via conventional methods.

In certain embodiments the measurement of multiple distinct areas is achieved as the wearable device comprises multiple optical fibres, each providing both an illumination path to direct the illuminating radiation to a distinct measurement area and a detection path to direct the reflected radiation to the spectrometer. This provides a low cost, straightforward means to measure the response of several different areas of the skin which may use a single light source and spectrometer

Preferably the spectrometer comprises a micro- or nano-spectrometer. Preferably it does not require active temperature stabilisation via cooling means (such as a fan or heat sink) but instead uses a factory temperature calibration to account for temperature changes during use. In this way the spectrometer is of reduced size and complexity for ease of integration into the wearable device and has a lower power requirement and longer lifetime given that it does not require cooling components such as a fan which are liable to malfunction and drain power. The spectrometer may include an integrated temperature sensor or use a temperature sensor within the wearable device to measure temperature to adjust output based on the factor calibration. The spectrometer may then be calibrated by using a spectral sensitivity function which is recorded at several stages in the expected operating temperature range within the framework of a factory temperature calibration step ($QE\lambda = f(T)$, measured with the temperature sensor, QE: quantum sensitivity, T: temperature). In later use of the modules, information about the current detector temperature is then collected and stored with each spectral measurement using the same integrated temperature sensor. The spectral measurement can then be calibrated for temperature using the associated stored temperature and the calibration model.

Preferably the spectrometer comprises an InGaAs detector array. Preferably the InGaAs detector is arranged in a ceramic package, preferably with the integrated temperature sensor inside.

The spectrometer preferably has a high thermal capacity so that it does not undergo rapid temperature changes during operation, in particular caused by the thermal power loss of a light source and the detection system. For example the spectrometer has a plastic housing (instead of a metal housing for example) so that the temperature detector does not change rapidly.

Preferably the spectrometer is a moldable monolithic spectrometer. All components of the spectrometer may be formed in a single chip. The spectrometer may be formed as a single flexible component such that it can be integrated into a wearable device and conform to the area of the skin.

Preferably the wearable device further comprises one or more sample illumination fibres arranged to direct near infrared radiation from the source to the measurement area of the skin and the reflected or transmitted near infrared radiation to the spectrometer; one or more reference fibres arranged to direct near infrared radiation from the source directly to the spectrometer; wherein the processing unit is configured to determine the output absorption spectrum based on the radiation received from the illumination fibres and the reference fibres. In this way, the spectrometer uses the source spectrum as an internal reference so the output absorption spectra can be adjusted to take into account changes in the source spectrum, for example due to temperature changes, current/voltage changes or aging of the source.

Preferably the reference fibres transmit a representative portion of the source radiation with respect to delivered spectral distribution, spatial distribution and delivered power.

Preferably the wearable device additionally comprises a shutter arranged to selectively switch between: allowing radiation from the illumination fibres to enter the spectrometer and blocking radiation from the reference fibres from entering the spectrometer; and allowing radiation from the reference fibres to enter the spectrometer and blocking radiation from the illumination fibres from entering the spectrometer. In this way, the wearable device can check the reference spectrum (corresponding to the source) when required, for example at regular predetermined intervals or if the system detects changes in system parameters by switching to select the source spectrum before returning to measuring the absorption spectrum of the skin. The micro shutter is preferably a mechanical micro shutter, for example a piezo- or electro-magnetic shutter.

In certain embodiments the reference fibre may additionally be arranged to direct radiation from the source to a photo sensor within the wearable device. In this way the characteristics of the radiation emitted by the source may be sensed with the photo sensor, for example, intensity, spatial distribution, spectral distribution etc. These sensed characteristics can be used to calibrate the spectrometer to ensure accurate absorption spectra are output.

Preferably the source of near-infrared radiation comprises an LED, preferably a single LED although in some example the device may include a plurality of LEDs. Using an LED as the source of NIR radiation provides low power consumption and longer device lifetime.

Preferably the LED is a broad band NIR LED. In some examples the LED is arranged to emit radiation over a wide range of the near infra-red spectrum than the portion of the spectrum sampled by the spectrometer. In some examples a broad band LED can be used which is arranged to illuminate over a limited wavelength range, for example 1400 to 1800 nm.

In a further aspect of the invention there is provided a method of estimating the blood glucose level of the user comprising: irradiating a measurement area of the skin of a user with a source of near-infrared radiation; receiving reflected or transmitted near infrared radiation with a spectrometer to determine an absorption spectrum comprising the absorbance intensities at a plurality of wavelengths, the wavelengths all within a portion of the near infrared spectrum having a substantially constant optical penetration depth; calculating an estimate of the blood glucose level of the wearer using a calibration model describing the relationship between near infrared absorption and blood glucose level.

Preferably the selected portion of the near infrared spectrum having a substantially constant optical penetration depth is in the range 1400 to 1800 nm.

Preferably the calibration model is determined by receiving measured absorption spectra from the spectrometer during a calibration period; receiving actual blood glucose level measurement data, recorded at regular intervals during the calibration period; fitting the measured absorption spectra to the actual glucose level measurement data to determine the calibration model describing the relationship between the near infrared absorption and blood glucose level

## BRIEF DESCRIPTION OF THE DRAWINGS

Embodiments of the invention will now be described, by way of example only, with reference to the accompanying drawings, in which:

Figure 1a schematically illustrates a wearable device for estimating the blood glucose level of the wearer according to the present invention;

Figure 1b schematically illustrates the sensor portion of a wearable device in contact with the skin of a wearer;

Figure 2 illustrates a spectrometer of the wearable device according to the present invention;

Figure 3a schematically illustrates the optical penetration depth of light;

Figure 3b schematically illustrates the absorption coefficients of water, glucose, fat and protein;

Figure 4 illustrates a method of using a wearable device according to the present invention;

Figure 5a illustrates one varaible from the pre-processed absorption spectra obtained during calibration of the of the device and measured glucose levels determined from invasive measurements;

Figure 5b illustrates the estimated glucose level determined by the wearable device according to the present invention.

## DETAILED DESCRIPTION

Near-infrared spectroscopy of glucose is based on measuring the amount of light reflected from, or absorbed by, tissue at specific wavelengths. As the glucose molecule ($C_6H_{12}O_6$) has a distinct light absorption profile in the NIR spectrum (as shown in Figure 3a), levels can be measured. NIR spectroscopy of glucose in practical applications is challenging, firstly because glucose spectrum profile overlaps with, and is distorted by, common tissue substances such as water, haemoglobin and lipids. Second, the overall result of a single NIR measurement is influenced by a range of other factors, including variations in blood pressure, temperature and local variations in the light-scattering properties of the skin. As a result the glucose profile is a very weak component in an overall NIR profile. Extracting the glucose profile from all other signal sources and distortions is possible in the current invention through using a portion of the NIR spectrum which has a low and constant skin penetration depth such that contributions from unwanted portions of tissue are minimised.

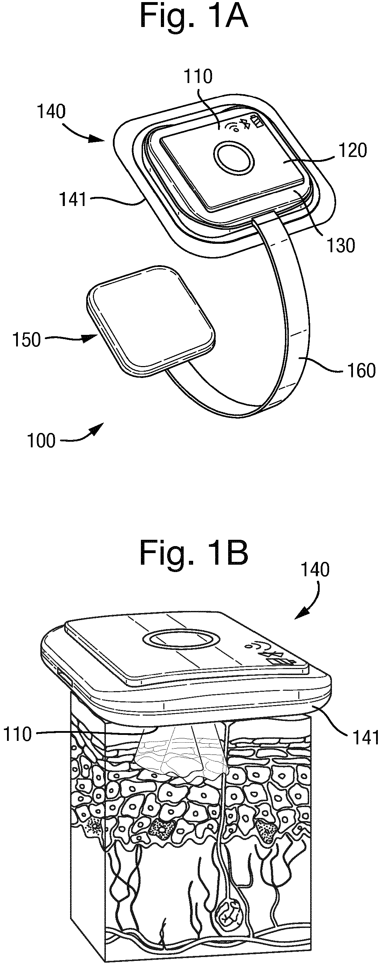

Figure 1a shows a wearable device 100 for estimating the blood glucose level of the wearer according to the present invention. The device 100 includes a source 110 of near infrared radiation arranged to irradiate a measurement area of the skin of the wearer. It also includes a spectrometer 120 arranged to perceive reflected or transmitted near infrared radiation and output an absorption spectrum comprising the absorbance intensities as a plurality of wavelengths, the wavelengths within a portion of the near infrared spectrum having a substantially constant optical penetration depth. The wearable device 100 also includes a processing unit 130 configured to receive the absorption spectrum from the spectrometer 120 and calculate an estimate of the blood glucose level of the wearer using a stored calibration model, the model describing the relationship between near infrared absorption and blood glucose level.

In the specific example of Figure 1 the source of near infrared radiation 110, the spectrometer 120 and the processing unit 130 are housed in a first housing 140. The device also comprises a second housing 150 which includes a rechargeable and replaceable battery. The first housing 140 and second housing 150 are connected together by a strap 160 which includes a power cable for connecting the battery to the components within the first housing 140. As shown in Figure 1, the first housing 140 has a substantially planar shape with a wide surface area portion configured to be placed against the skin of the user. The source 110 is arranged such that it irradiates the skin of the user when the skin interfacing broad surface area side 141 is brought into contact with the wearer's skin. The housing 140 and particularly the skin engaging side 141 may be flexible such that it can deform to conform to the skin shape of a user.

The components of the wearable device may be combined into any form of wearable device, for example a watch or wristband or item of clothing so long as the skin interfacing surface including the optical path along which the irradiating light is directed is held against the skin of the user. Figure 1b shows the first housing 140, containing the light source 110, spectrometer 120 and processing unit 130, held against the skin of the user in use. As shown the source 110 of NIR radiation is arranged such that a portion of the skin under the device is irradiated. The first housing 140 has a substantially flat form factor meaning the first housing 140, housing the sensing components, is held close to the skin, minimising obstruction or irritation to the user.

In the specific example of Figure 1, the sensor is attached to the upper arm by half-circle flexible hoop 160, which naturally balances the weight of the sensor components in the first housing 140 with the battery in the second housing 150. This arrangement makes the wearable device much lighter, more discrete and much easier to wear (as it can easily be worn under clothes). The output reading can be transmitted to an external device for viewing, for example, on the user's smartphone or smartwatch. This arrangement reduces both production complexity and cost of production.

The source of near infrared radiation 110 preferably comprises an LED such that the wearable device benefits from the low power consumption and longer device lifetime of such devices. The device 100 uses a hollow waveguide such as an optical fibre (not shown) to direct the emitted light from the LED 110 to a measurement area of the wearer's skin. In some examples, multiple fibres are used to direct the light to a number of distinct areas of the skin, thereby allowing a larger representative skin area to be sampled by the device.

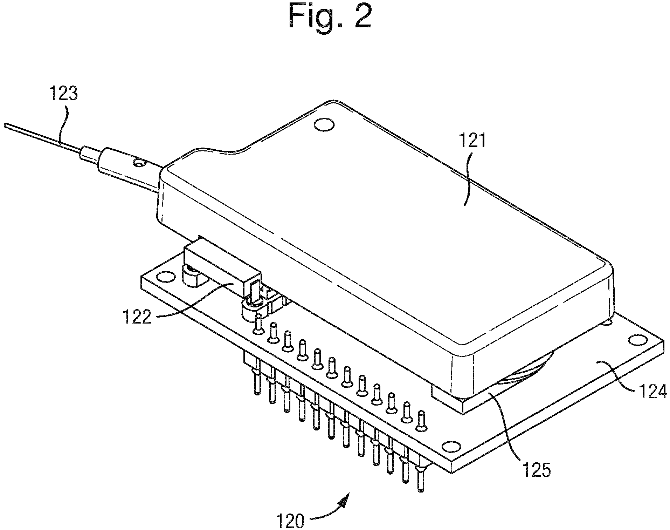

Figure 2 schematically illustrates a spectrometer 120 used with the wearable device of the present invention. The spectrometer 120 is a near infrared micro spectrometer. The micro spectrometer 120 includes a housing 121 and an indium gallium arsenide detector array 122. The spectrometer 120 further includes an optical fibre 123 arranged to direct the reflected or transmitted near infrared radiation to the indium gallium arsenide detector array 122. Unlike conventional spectrometers, the spectrometer 120 in this example incorporates a temperature sensor in order to calibrate the spectrometer based on a measured temperature, rather than including active cooling components such as a fan and this further means that the power consumption of the spectrometer 120 is far below that of conventional devices meaning the overall power consumption of the wearable device 100 is significantly reduced. Instead of using active cooling components to keep the temperature of the detector within a specific working range, the spectrometer is calibrated prior to use by recording a spectral sensitivity function at several different temperatures in the expected operating temperature range. This may be carried out with a dedicated integral temperature sensor to the spectrometer 120 or with the temperature sensor of the wearable device which is also used to sense the skin temperature of the user.

During use of the spectrometer the detected temperature is measured and stored with each spectral measurement. The measured spectrum can then be calibrated based on the measured temperature using the temperature calibration data set collected during calibration of the spectrometer 120. In this way, no active cooling components are required within the spectrometer or wearable device housing. This allows the size of the spectrometer to be reduced significantly and dispenses with the need for any hermetically sealed housing reducing the cost of the system and allows the spectrometer 120 to be incorporated in a single chip such that it is readily applied in the wearable device of the current invention. The spectrometer 120 may further comprise a substrate 124 onto which the various components of the spectrometer 120 are incorporated. The substrate may be flexible such that it can be incorporated in a flexible patch or planar housing such as the first housing 140 of the wearable device 100. The spectrometer may further include data memory or processing capability 125 although these components may also alternatively be provided as the processing unit of the wearable device 100.

The spectrometer 120 is arranged to determine the intensity of the received near infrared radiation at a plurality of wavelengths over a portion of the near infrared spectrum having a substantially constant optical penetration depth in the tissue of the user. In particular, the spectrometer is configured to determine the intensity of received near infrared radiation at a plurality of wavelengths in the range 1400 to 1800 nanometres.

As shown in Figure 3a, in this range of the near infrared spectrum the optical penetration depth is substantially constant. In particular it remains between approximately 1 and 1.5 mm. Therefore, wavelengths in this range penetrate a small and constant distance into the skin of the user. The optical paths travelled by the instant radiation are therefore fairly similar such that the wearable device 100 samples a specific volume of the user's tissue. As schematically illustrated in Figure 1b the volume of the tissue sensed by the sensor is small and relatively constant such that the signal produced relates to a known reproducible and constant volume. This reduces motion artefacts introduced by movement of the body while the wearable device is attached to the user, as the maximum penetration depth is much reduced and fairly constant such that changes in orientation do not produce significant changes in the portion of the tissue being sampled. Furthermore, as shown in Figure 3b, this portion of the spectrum relates to a peak in the absorption spectrum of glucose, thereby providing a good signal to noise ratio in the absorption spectrum relating to the glucose.

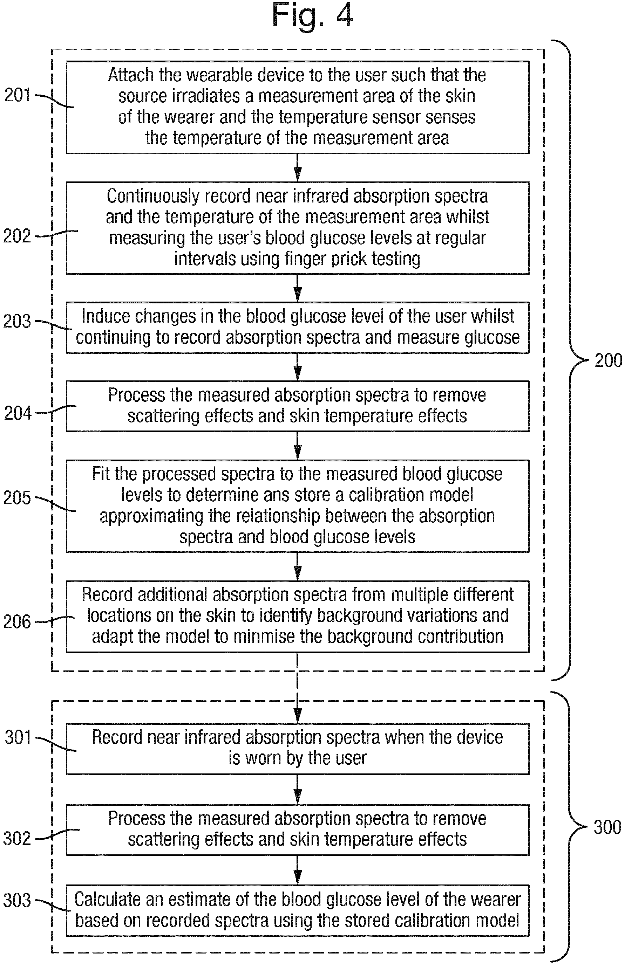

Figure 4 illustrates a method of using the wearable device according to the present invention. The method is split into two sections, the first part of the method 200 relates to calibration of the wearable device, the second part 300 relates to use of the wearable device to determine a blood glucose level of the wearer. In some examples of the device, the calibration method 200 may be carried out for each specific user such that the calibration model determined is specific to that user. In other examples of the device, the calibration method 200 may be carried out across large numbers of users in order to develop a global calibration model such that the device does not need to be calibrated to a specific user.

In the first step 201 of the calibration method 200 the wearable device is attached to a user such that the source 110 irradiates a measurement area of the user's skin and the temperature sensor senses a temperature of the measurement area of the skin. The wearable device 100 may for example be a wristband or patch arranged to attach to the forearm of a user. The wearable device is attached such that it remains in a fairly constant position. The attachment means may be a strap which can be tightened or an adhesive patch which attaches to the skin. In this way the measurement area irradiated by the source remains fairly constant.

In step 202 the device starts recording the near infrared absorption spectra with the spectrometer and records the temperature of the measurement area of the skin. Simultaneously, the user's blood glucose levels are measured at regular intervals using conventional invasive testing such as finger prick testing. The finger prick testing may be provided as a complementary device and can communicate with the wearable device 100 to provide the measured blood glucose levels of the user automatically, for example by wireless or cabled connection. Alternatively a user may input and be measured blood glucose levels into the device manually.

At step 203, whilst continuously recording the absorption spectra and a temperature whilst measuring the user's actual blood glucose level by conventional means, changes are induced in the user's blood glucose level in order to provide a greater range of output data with which to construct the calibration model. The changes may be induced by the user consuming glucose, for example via food or drink or carrying out certain exercises to reduce the blood glucose levels.

At step 204 the measured absorption spectra are processed in order to remove potential sources of error. In particular, scattering effects are removed by calculating the second derivative of the spectra. This process may be carried out by the processing unit of the device as the data is gathered or once the complete data set has been obtained. Skin temperature effects are also removed using latent structure decomposition methods. In particular, skin temperature effects may be removed using marker variable projection techniques which use as an input the temperature of the skin measurement area sensed by the temperature sensor. In general such methods involve projecting the multivariable data onto latent variables to determine the contribution of the skin temperature and accordingly the skin temperature effects can be removed. At step 205 the processed spectra are fitted to the measured actual blood glucose levels to determine and store a calibration model which approximates the relationship between the absorption spectra and the blood glucose level. The specific fitting and determining of the model may be carried out by any suitable known method, for example using principle component regression, partial least squares regression or other modelling approaches based on latent variables.

At step 206, additional absorption spectra are recorded from multiple different locations on the skin to identify background variations and adapt the model to minimise contribution of these background effects. In particular, the specific absorption response will vary depending on the specific location, for example due to the specific composition of tissue in that area. Therefore, if the devices calibrated for a specific position on the skin and then moves later on during use, an offset will be introduced to the absorption spectra. By adapting the model to take into account the varied spectra in multiple different locations on the skin, these background variations can be minimised.

At the end of the calibration method 200 therefore, a calibration model is determined which can provide a close estimate of the blood glucose level based on the measured near infrared absorption spectra.

The second part of the method 300 follows the calibration, once the calibration model has been determined. The wearable device can then be used at any point by the user to determine the blood glucose levels.

At step 301 the wearable device is worn by the user and switched on to begin irradiating a measurement area of the skin with the near infrared radiation and collecting the infrared absorption spectra in a region of the spectrum in which the optical penetration depth is substantially constant, mainly for example 1400 to 1800 nanometres. The absorption spectrum preferably includes at least 10 wavelengths over the range 1400 to 1800 nanometres in order to improve the accuracy in which the calibration model can estimate the blood glucose level.

At step 302 the measured absorption spectra are pre-processed to remove scattering effects and skin temperature effects, as described under step 204.

At step 303 the processing unit uses the stored calibration model to calculate an estimate of the blood glucose level of the wearer, based on the received recorded spectra. In this way, the wearable device may provide a continuous estimate of the wearer's blood glucose level. The device should not require unnecessary follow up calibrations however a safety function may be implemented in the wearable device 100 whereby if the device senses exceptional circumstances the user may be prompted to provide a manual glucose reading such that the device can recalibrate. For example, if the estimated glucose level reaches the boundary of a predefined window, for example outside of a range tested during calibration, the user may be prompted to recalibrate the device. Measurements may be transferred wirelessly to the user's smartphone or smart watch where they can be displayed, for example on the cover page. Measurements may also be transferred to a cloud storage service such that historical data may be accessed by the user and, optionally, medical staff. The device can also provide an alarm if glucose levels become too high or too low.

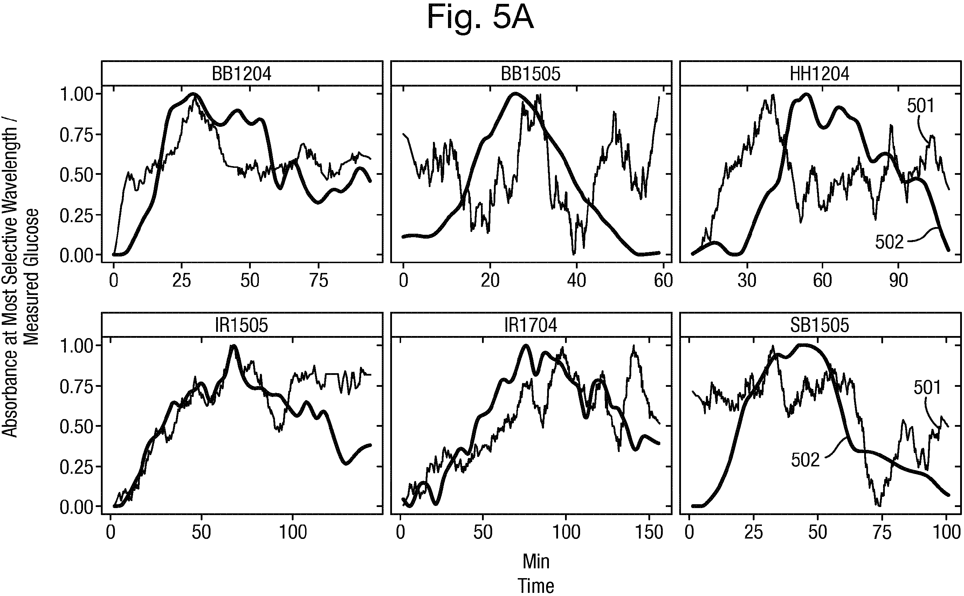

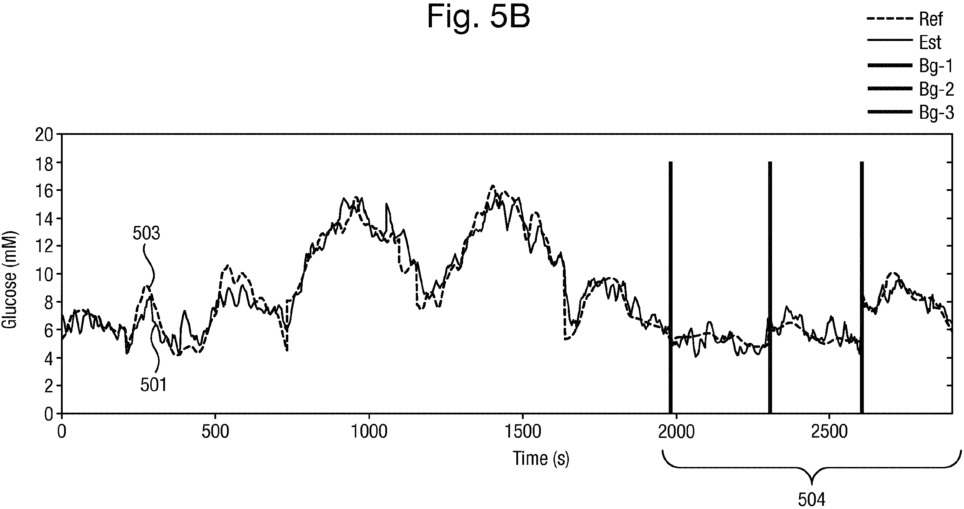

Figure 5a illustrates data collected during the calibration method 200. In particular, calibration data from six calibration sessions are shown in which the actual blood glucose level 502 is shown against the pre-processed absorption data 501 at the most selective wavelength range for glucose, corresponding to the portion of the near infrared spectrum having a substantially constant optical penetration depth. The absorption spectra 501 have been pre-processed to remove scattering effects using the second derivative of the spectra and removing skin temperature effects using marker variable projections. Figure 5b illustrates the estimated glucose level 503 predicted using the calibration model based on the data of Figure 5a. The last three segments 504 relate to measurements at random location on the forearm and are used for including natural background variations into the calibration model. The absorption spectra naturaly varies depending on the location on the body the device is worn due to the specific composition of the various different tissue types in that area. By including data from multiple areas of the body, these background contributions can be isolated and substantially removed, providing better signal to noise. As shown in Figure 3b the calibration model is able to accurately estimate the blood glucose level of the wearer of the device based on the received absorption spectra.

With the wearable device of the present invention, a non-invasive measurement of blood glucose may be determined in which errors due to motion artefacts and the contribution due to multiple tissue types and changing sampling area are significantly reduced.

**Claims**

1. A wearable device for estimating the blood glucose level of the wearer, the device comprising:

    a source of near-infrared radiation arranged to irradiate a measurement area of the skin of the wearer;

a spectrometer arranged to receive reflected or transmitted near infrared radiation and output an absorption spectrum comprising the absorption intensity at a plurality of wavelengths, wherein all of the wavelengths are within a portion of the near infrared spectrum having a substantially constant optical penetration depth; a processing unit configured to:

receive the absorption spectrum from the spectrometer;
calculate an estimate of the blood glucose level of the wearer using a stored calibration model describing the relationship between near infrared absorption and blood glucose level.

2. The wearable device of claim 1 wherein the wavelengths of the output absorption spectrum are all within the range 1400 to 1800 nm.

3. The wearable device of claim 2 wherein the absorption spectrum comprises at least 10 wavelengths in the range 1400 to 1800 nm.

4. The wearable device of any preceding claim further comprising:

a temperature sensor arranged to measure the temperature of the measurement area of the skin of the wearer;
wherein the processing unit is further configured to:

receive the temperature from the temperature sensor; and
adjust the absorption spectra by using marker variable projections based on the measured temperature to remove skin temperature effects.

5. The wearable device of any preceding claim wherein the processing unit is further configured to:

remove the effects of scattering in the measured intensities received from the spectrometer.

6. The wearable device of claim 5 wherein the processing unit is configured to remove effects of scattering by calculating a second derivative of the absorption spectrum.

7. The wearable device of any preceding claim wherein the processing unit is configured to determine the calibration model by carrying out a calibration routine comprising:

receiving measured absorption spectra from the spectrometer during a calibration period;
receiving actual blood glucose level measurement data, recorded at regular intervals during the calibration period;
fitting the measured absorption spectra to the actual glucose level measurement data to determine the calibration model describing the relationship between the near infrared absorption and blood glucose level.

8. The wearable device of claim 7 wherein the absorption spectra received during the calibration period are collected from a plurality of distinct locations on the skin of the wearer and the processing unit is further configured to use the spectra from distinct areas to adapt the calibration model to reduce the component of the spectra related to natural background variations.

9. The wearable device of any preceding claim configured to direct the illuminating near-infrared radiation to multiple distinct measurement areas of the skin, the spectrometer arranged to receive the reflected or transmitted near infrared radiation from the multiple areas to determine an average absorption spectrum from the multiple distinct measurement areas.

10. The wearable device of claim 9 further comprising multiple optical fibres, each providing both an illumination path to direct the illuminating radiation to a distinct measurement area and a detection path to direct the reflected radiation to the spectrometer.

11. The wearable device of any preceding claim wherein the spectrometer is a moldable monolithic spectrometer.

12. The wearable device of any preceding claim wherein the spectrometer comprises a temperature sensor and the spectrometer is calibrated to adjust the output spectra dependent on the sensed temperature.

13. The wearable device of any preceding claim wherein the source of near-infrared radiation comprises a near-infrared emitting LED, preferably arranged to emit radiation over a wider range of the near infra-red spectrum than the portion of the spectrum sampled by the spectrometer.

14. The wearable device of any preceding claim further comprising:

one or more sample illumination fibres arranged to direct near infrared radiation from the source to the measurement area of the skin and direct the reflected or transmitted near infrared radiation to the spectrometer;
one or more reference fibres arranged to direct near infrared radiation from the source directly to the spectrometer;
wherein the processing unit is configured to determine the output absorption spectrum based on the radiation received from the illumination fibres and the reference fibres.

15. The wearable device of claim 14 further comprising:

a shutter arranged to selectively switch between:

a first position, allowing radiation from the illumination fibres to enter the spectrometer and blocking radiation from the reference fibres from entering the spectrometer; and
a second position, allowing radiation from the reference fibres to enter the spectrometer and blocking radiation from the illumination fibres from entering the spectrometer

# Fig. 1A

# Fig. 1B

# Fig. 2

# Fig. 3A

### Optical Penetration Depth of Light

$$\delta = \frac{1}{\sqrt{3\mu_a(\mu_a + \mu'_s)}}$$

# Fig. 3B

### Absorption Coefficients

# Fig. 4

201 — Attach the wearable device to the user such that the source irradiates a measurement area of the skin of the wearer and the temperature sensor senses the temperature of the measurement area

202 — Continuously record near infrared absorption spectra and the temperature of the measurement area whilst measuring the user's blood glucose levels at regular intervals using finger prick testing

203 — Induce changes in the blood glucose level of the user whilst continuing to record absorption spectra and measure glucose

204 — Process the measured absorption spectra to remove scattering effects and skin temperature effects

205 — Fit the processed spectra to the measured blood glucose levels to determine ans store a calibration model approximating the relationship between the absorption spectra and blood glucose levels

206 — Record additional absorption spectra from multiple different locations on the skin to identify background variations and adapt the model to minmise the background contribution

200

301 — Record near infrared absorption spectra when the device is worn by the user

302 — Process the measured absorption spectra to remove scattering effects and skin temperature effects

303 — Calculate an estimate of the blood glucose level of the wearer based on recorded spectra using the stored calibration model

300

Fig. 5A

Fig. 5B

EP 3 636 141 A1

Europäisches
Patentamt

European
Patent Office

Office européen
des brevets

# EUROPEAN SEARCH REPORT

**Application Number**

EP 18 19 9737

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/173255 A1 (ACOSTA GEORGE M [US] ET AL) 3 August 2006 (2006-08-03) <br> * paragraph [0081] - paragraph [0096] * <br> * paragraph [0104] - paragraph [0108] * <br> * paragraph [0120] - paragraph [0124] * <br> * paragraph [0133] - paragraph [0138] * <br> ----- | 1,4-7, 11-13 | INV. <br> A61B5/00 <br> A61B5/145 <br> A61B5/1455 <br> A61B5/1495 <br> G01N21/35 <br> G01N33/66 |
| X | US 2018/146899 A1 (LEE SO YOUNG [KR] ET AL) 31 May 2018 (2018-05-31) <br> * figures 1, 2 * <br> * paragraph [0057] - paragraph [0076] * <br> * paragraph [0085] - paragraph [0095] * <br> * paragraph [0107] - paragraph [0115] * <br> * paragraph [0129] - paragraph [0134] * <br> ----- | 1,7,8, 11,13 | |
| X | LHOUCINE BEN MOHAMMADI ET AL: "In vivo evaluation of a chip based near infrared sensor for continuous glucose monitoring", BIOSENSORS AND BIOELECTRONICS, vol. 53, 1 March 2014 (2014-03-01), pages 99-104, XP055542754, AMSTERDAM, NL ISSN: 0956-5663, DOI: 10.1016/j.bios.2013.09.043 <br> * figures 1, 3 * <br> * abstract * <br> * page 99 - page 104 * <br> ----- | 1-3,7, 11,13-15 | |
| | | | **TECHNICAL FIELDS SEARCHED (IPC)** |
| A | WO 96/17546 A1 (OPTISCAN INC [US]) 13 June 1996 (1996-06-13) <br> * claim 1 * <br> ----- | 4,12 | A61B <br> G01N |
| A | WO 02/38043 A2 (ARGOSE INC [US]; MANSFIELD JAMES [US]) 16 May 2002 (2002-05-16) <br> * figures 1-3 * <br> * page 10, line 14 - page 11, line 10 * <br> ----- | 8-10 | |

The present search report has been drawn up for all claims

| Place of search | Date of completion of the search | Examiner |
|---|---|---|
| The Hague | 23 January 2019 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 636 141 A1**

## ANNEX TO THE EUROPEAN SEARCH REPORT
## ON EUROPEAN PATENT APPLICATION NO.

EP 18 19 9737

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2019

| Patent document cited in search report | | | Publication date | Patent family member(s) | | | Publication date |
|---|---|---|---|---|---|---|---|
| US 2006173255 | A1 | | 03-08-2006 | AU | 2003218012 | A1 | 22-09-2003 |
| | | | | CA | 2476421 | A1 | 18-09-2003 |
| | | | | CN | 1638690 | A | 13-07-2005 |
| | | | | EP | 1499231 | A2 | 26-01-2005 |
| | | | | JP | 4189322 | B2 | 03-12-2008 |
| | | | | JP | 2005519682 | A | 07-07-2005 |
| | | | | MX | PA04008713 | A | 24-02-2006 |
| | | | | TW | I284200 | B | 21-07-2007 |
| | | | | US | 2005010090 | A1 | 13-01-2005 |
| | | | | US | 2006116562 | A1 | 01-06-2006 |
| | | | | US | 2006173254 | A1 | 03-08-2006 |
| | | | | US | 2006173255 | A1 | 03-08-2006 |
| | | | | US | 2006183983 | A1 | 17-08-2006 |
| | | | | US | 2006195023 | A1 | 31-08-2006 |
| | | | | US | 2006211927 | A1 | 21-09-2006 |
| | | | | WO | 03076883 | A2 | 18-09-2003 |
| US 2018146899 | A1 | | 31-05-2018 | CN | 108113683 | A | 05-06-2018 |
| | | | | EP | 3329848 | A1 | 06-06-2018 |
| | | | | KR | 20180061959 | A | 08-06-2018 |
| | | | | US | 2018146899 | A1 | 31-05-2018 |
| WO 9617546 | A1 | | 13-06-1996 | AU | 697835 | B2 | 15-10-1998 |
| | | | | CA | 2206116 | A1 | 13-06-1996 |
| | | | | EP | 0901339 | A1 | 17-03-1999 |
| | | | | JP | 3590409 | B2 | 17-11-2004 |
| | | | | JP | H10510180 | A | 06-10-1998 |
| | | | | US | 5615672 | A | 01-04-1997 |
| | | | | WO | 9617546 | A1 | 13-06-1996 |
| WO 0238043 | A2 | | 16-05-2002 | AU | 3042902 | A | 21-05-2002 |
| | | | | US | 2002058864 | A1 | 16-05-2002 |
| | | | | WO | 0238043 | A2 | 16-05-2002 |

EPO FORM P0459

For more details about this annex : see Official Journal of the European Patent Office, No. 12/82

**REFERENCES CITED IN THE DESCRIPTION**

**Patent documents cited in the description**

- WO 2017129633 A

- WO 2017129634 A

**Non-patent literature cited in the description**

- **O. M KVALHEIM.** Latent-Structure Decompositions (Projections) of Multivariate Data. *Chemometrics and Intelligent Laboratory Systems,* 1987, vol. 2, 283-290